# FASCICULE DE BREVET EUROPEEN

(11) **EP 0 723 775 B1**
(45) Date de publication et mention de la délivrance du brevet: **10.12.1997**
(21) Numéro de dépôt: 96400049.1
(22) Date de dépôt: 09.01.1996
(51) Int. Cl.: A61K 7/48

(54) **Utilisation du sulfate de déhydroépi-androstérone dans une composition cosmétique ou dermatologique**
Anwendung von Dehydroepi-androsteronsulfat in einer kosmetischen oder dermatologischen Zubereitung
Use of dehydroepi-androsterone in cosmetic or dermatologic composition

(30) Priorité: 26.01.1995 FR 9500899
(43) Date de publication de la demande: 31.07.1996
(73) Titulaire: L'OREAL, 75008 Paris (FR)
(72) Inventeur: Breton, Lionel, F-78000 Versailles (FR); Lacharriere, Olivier de, F-75015 Paris (FR)
(74) Mandataire: Lhoste, Catherine

(56) Documents cités:
- EP-A- 0 189 738
- WO-A-94/16709
- FR-A- 2 405 068
- CHEMICAL ABSTRACTS, vol. 104, no. 2, 13 Janvier 1986, Columbus, Ohio, US; abstract no. 10399s, page 302 ;colonne G ; & JP-A-60 161 912 (KANEBO, LTD)

## Description

L' invention se rapporte à l'utilisation du 3-sulfate de déhydroiso-androstérone de sodium, aussi connue sous le nom de sulfate de déhydroépi-androstérone et sous l'abréviation S-DHEA, dans une composition cosmétique ou dermatologique à application topique, destinée au traitement de certains signes du vieillissement endogène et/ou exogène.

Le vieillissement cutané résulte des effets sur la peau de facteurs intrinsèques et extrinsèques. Cliniquement, les signes du vieillissement se traduisent par l'apparition de rides et ridules, par un relâchement des tissus cutanés et sous-cutanés, par une perte de l'élasticité cutanée, par une atonie de la texture de la peau, et par le jaunissement de la peau qui devient plus terne et sans éclat. Sur les zones de la peau qui ont été exposées au soleil tout au long de la vie - essentiellement le visage, le décolleté, les mains et les avant bras - on observe souvent des tâches pigmentaires, des télangiectasies et une élastose.

Certains de ces signes sont plus particulièrement liés au vieillissement intrinsèque ou physiologique, c'est-à-dire au vieillissement lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

Les changements de la peau résultant du vieillissement intrinsèque ou physiologique sont la conséquence d'une sénescence génétiquement programmée où interviennent des facteurs endogènes. Ce vieillissement intrinsèque provoque notamment un ralentissement du renouvellement des cellules de la peau. Histologiquement la peau est globalement amincie, tant au niveau épidermique que dermique. La densité des macromolécules fibreuses du derme (élastine et collagène) est diminuée. Au contraire, le vieillissement extrinsèque entraîne des changements histopathologiques tels qu'une excessive accumulation de matière élastique dans le derme supérieur et une dégénérescence des fibres de collagène.

L'invention s'intéresse essentiellement aux rides, ridules, au relâchement des tissus cutanés et sous-cutanés et à l'éclat de la peau. Le relâchement des tissus cutanés et sous-cutanés se traduit par une texture de peau atone, un relâchement du microrelief cutané, une fermeté cutanée diminuée, et une peau globalement flasque.

On connait de nombreuses compositions qui prétendent traiter les rides et les ridules de la peau ou raffermir les tissus cutanés, mais ces compositions ne traitent qu'incomplétement et temporairement ces désordres morphologiques. Aussi, il subsiste le besoin d'une composition à application topique permettant de traiter plus efficacement les rides et les ridules ainsi que de raffermir les tissus cutanés. L'invention permet, de plus, de donner un éclat à la peau âgée, comparable à celui d'une peau plus jeune.

L'invention a donc pour objet l'utilisation du sulfate de déhydroépi-androstérone dans et/ou pour la préparation d'une composition topique pour traiter les rides, les ridules et/ou lutter contre le relâchement cutané et/ou sous-cutané et/ou raviver l'éclat de la peau.

Plus particulièrement, l'invention se rapporte à l'utilisation du sulfate de déhydroépi-androstérone dans et/ou pour la préparation d'une composition topique pour traiter le relâchement et/ou l'avachissement (ou l'effondrement) du microrelief cutané, la flaccidité cutanée et/ou sous-cutanée et/ou raffermir la peau et/ou tonifier la texture de la peau.

L'invention a aussi pour objet un procédé de traitement cosmétique des rides et/ou des ridules et/ou du relâchement cutané et/ou sous-cutané en vue de raffermir la peau et/ou pour raviver l'éclat de la peau, comprenant l'application sur la peau d'une composition cosmétique contenant du sulfate de déhydroépi-androstérone.

Certes, il est connu par les documents EP-A-189783 et JP-A-60161912 des compositions topiques contenant du sulfate de déhydroépi-androstérone pour traiter la peau sèche ou rêche. En outre, il est connu par le document WO-A-94/16709 d'utiliser le sulfate de déhydroépi-androstérone, y compris par voie transdermique, pour pallier aux insuffisances hormonales. Toutefois, ces documents n'enseignent pas d'utiliser le sulfate de déhydroépi-androstérone pour traiter les rides et/ou ridules ou le relâchement cutané et/ou sous-cutané.

La composition contient un milieu cosmétiquement ou dermatologiquement acceptable, c'est-à-dire compatible avec les tissus cutanés. Ainsi, la composition peut être appliquée sur tout le corps humain.

Le S-DHEA est utilisé à titre d'exemple en une quantité allant de 0,00001 % à 5 % du poids total de la composition, de préférence de 0,0001 % à 1 % et mieux de 0,001 % à 0,5 %.

Les compositions utilisables dans l'invention peuvent se présenter sous toutes les formes galéniques classiquement utilisées pour une application topique et notamment sous forme de solutions aqueuses, hydroalcooliques ou huileuses, de dispersions du type lotion ou sérum, de gels anhydres ou lipophiles, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition cosmétique ou dermatologique de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Comme actifs lipophiles ou hydrophiles utilisables dans l'invention en vue de parfaire le traitement des rides, ridules, la lutte contre le relâchement cutané et/ou sous-cutané et/ou l'éclat de la peau, on peut citer par exemple les rétinoïdes (rétinol et ses esters, rétinal, acide rétinoïque et ses dérivés, rétinoïdes, notamment ceux décrits dans les documents FR-A-2 570 377, EP-A-199636, EP-A-325540, EP-A-402072), les α-hydroxy-acides (glycolique, lactique, malique, citrique, tartrique, mandélique), les β-hydroxy-acides (acide salicylique et ses dérivés notamment alcoylés), les α-céto-acides, les β-céto-acides, les peroxydes comme le peroxyde de benzoyle, les vitamines notamment E, F, les actifs anti-radicaux comme la superoxyde-dismutase, le sélénium, le zinc, les béta-carotènes.

La composition peut en outre contenir d'autres hormones que le S-DHEA, naturelles ou synthétiques, oestrogéniques, progestatives ou androgéniques comme la progestérone, la testostérone, l'oestradiol anhydre, le broparestrol, l'oestrone, l'acétate de prégnénolone, la prégnénolone, la 17 béta-hydroxyprogestérone, le propionate de testostérone, l'androstènedione et les androstanediols.

Les exemples suivants illustrent l'invention. Dans ces exemples, les proportions indiquées sont des pourcentages en poids.

### Exemple 1 : Crème pour lutter contre le relâchement des tissus cutanés (émulsion huile-dans-eau)

| | |
|---|---|
| S-DHEA de Na | 0,05 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Huile de germe de maïs | 12,00 |
| Huile de tournesol | 10,00 |
| Antioxydant | 0,05 |
| Parfum | 0,50 |
| Conservateur | 0,30 |
| Eau | qsp 100 % |

### Exemple 2 : Crème de soin antirides pour le visage (émulsion huile-dans-eau)

| | |
|---|---|
| S-DHEA de Na | 0,10 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Huile de tournesol | 12,00 |
| Huile siliconée | 12,00 |
| Antioxydant | 0,05 |
| Parfum | 0,50 |
| Conservateur | 0,30 |
| Eau | qsp 100 % |

### Exemple 3 : Crème pour redonner de l'éclat à une peau vieillie

| | |
|---|---|
| S-DHEA de Na | 0,03 |
| Estrone | 0,005 |
| Estradiol | 0,005 |
| Acétate de prégnénolone | 0,01 |
| Huile de ricin hydrogénée | 12,00 |
| Huile de tournesol | 8,00 |
| Polysorbate 60 | 1,00 |
| Stéarate de glycérol | 2,00 |
| Carbomer | 0,40 |
| Triéthanolamine | 0,70 |
| Antioxydant | 0,05 |
| Parfum | 0,05 |
| Conservateur | 0,30 |
| Eau | qsp 100 % |

## Revendications

1. Utilisation cosmétique du sulfate de déhydroépi-androstérone dans une composition topique pour traiter les rides, les ridules et/ou lutter contre le relâchement cutané et/ou sous-cutané et/ou raviver l'éclat de la peau et/ou pour traiter le relâchement et/ou l'effondrement du microrelief cutané, la flaccidité cutanée et/ou sous-cutanée et/ou raffermir la peau et/ou tonifier la texture de la peau.

2. Utilisation du sulfate de déhydroépi-androstérone pour la préparation d'une composition topique thérapeutique pour traiter les rides, les ridules et/ou lutter contre le relâchement cutané et/ou sous-cutané et/ou raviver l'éclat de la peau et/ou pour traiter le relâchement et/ou l'effondrement du microrelief cutané, la flaccidité cutanée et/ou sous-cutanée et/ou raffermir la peau et/ou tonifier la texture de la peau.

3. Utilisation selon la revendication 1 ou 2, caracterisée en ce que la composition contient, en outre, au moins un actif choisi parmi les alpha-hydroxyacides, les béta-hydroxyacides, les alpha-cétoacides, les béta-cétoacides, les rétinoïdes, le peroxyde de benzoyle, les anti-radicaux libres.

4. Utilisation selon l'une des revendications 1 à 3, caractérisée en ce que la composition contient, de plus, au moins une hormone choisie parmi les hormones naturelles et de synthèse, oestrogéniques, progestatives et androgéniques.

5. Procédé de traitement cosmétique des rides et/ou ridules et/ou du relâchement cutané et/ou sous-cutané et/ou pour raviver l'éclat de la peau, comprenant l'application sur la peau d'une composition cosmétique contenant du sulfate de déhydroépi-androstérone.

## Claims

1. Cosmetic use of dehydro-epi-androsterone sulphate in a topical composition for treating wrinkles and fine lines and/or for combating cutaneous and/or subcutaneous slackening and/or for reviving the radiance of the skin and/or for treating the slackening and/or collapse of the cutaneous microrelief, cutaneous and/or subcutaneous flaccidity, and/or for firming up the skin and/or for toning up the texture of the skin.

2. Use of dehydro-epi-androsterone sulphate for the preparation of a therapeutic topical composition for treating wrinkles, fine lines and/or for combating cutaneous and/or subcutaneous slackening and/or for reviving the radiance of the skin and/or for treating the slackening and/or collapse of the cutaneous microrelief, cutaneous and/or subcutaneous flaccidity, and/or for firming up the skin and/or for toning up the texture of the skin.

3. Use according to Claim 1 or 2, characterized in that the composition also contains at least one active agent chosen from alpha-hydroxy acids, beta-hydroxy acids, alpha-keto acids, beta-keto acids, retinoids, benzoyl peroxide and anti-free-radical agents.

4. Use according to one of Claims 1 to 3, characterized in that the composition furthermore contains at least one hormone chosen from natural and synthetic oestrogenic, progestative and androgenic hormones.

5. Process for the cosmetic treatment of wrinkles and/or fine lines and/or of cutaneous and/or subcutaneous slackening and/or for reviving the radiance of the skin, comprising the application of a cosmetic composition containing dehydro-epi-androsterone sulphate to the skin.

## Patentansprüche

1. Kosmetische Verwendung von Dehydroepiandrosteronsulfat in einer topischen Zusammensetzung zur Behandlung von Falten und Fältchen und/oder zur Bekämpfung der Erschlaffung der Haut und/oder des Unterhautgewebes und/oder zur Belebung des Glanzes der Haut und/oder zur Behandlung der Erschlaffung und/oder des Verlustes des Mikroreliefs der Haut, der Schlaffheit der Haut und/oder des Unterhautgewebes und/oder zur Straffung der Haut und/oder zur Stärkung der Textur der Haut.

2. Verwendung von Dehydroepiandrosteronsulfat zur Herstellung einer therapeutischen topischen Zusammensetzung zur Behandlung von Falten und Fältchen und/oder zur Bekämpfung der Erschlaffung der Haut und/oder des Unterhautgewebes und/oder zur Belebung des Glanzes der Haut und/oder zur Behandlung der Erschlaffung und/oder des Verlustes des Mikroreliefs der Haut, der Schlaffheit der Haut und/oder des Unterhautgewebes und/oder zur Straffung der Haut und/oder zur Stärkung der Textur der Haut.

3. Verwendung nach Anspruch 1 oder 2, dadurch gekennzeichnet, daß die Zusammensetzung ferner mindestens einen Wirkstoff enthält, der unter den α-Hydroxysäuren, β-Hydroxysäuren, α-Ketosäuren, β-Ketosäuren, Retinoiden, Benzoylperoxid und Mitteln gegen freie Radikale ausgewählt ist.

4. Verwendung nach einem der Ansprüche 1 bis 3, dadurch gekennzeichnet, daß die Zusammensetzung ferner mindestens ein Hormon enthält, das unter den natürlichen und synthetischen Hormonen vom Typ der Östrogene, Gestagene und Androgene ausgewählt ist.

5. Verfahren zur kosmetischen Behandlung von Falten und/oder Fältchen und/oder der Erschlaffung der Haut und/oder des Unterhautgewebes und/oder zur Belebung des Glanzes der Haut, das das Auftragen einer kosmetischen Zusammensetzung auf die Haut umfaßt, welche Dehydroepiandrosteronsulfat enthält.
